(19) Europäisches Patentamt
European Patent Office
Office européen des brevets

(11) **EP 3 391 809 A1**

(12) **EUROPEAN PATENT APPLICATION**

(43) Date of publication:
**24.10.2018 Bulletin 2018/43**

(51) Int Cl.:
*A61B 5/00* (2006.01)  *A61B 5/083* (2006.01)
*A61B 5/08* (2006.01)  *A61B 5/11* (2006.01)
*A61B 5/0205* (2006.01)  *A61B 5/024* (2006.01)
*A61B 5/053* (2006.01)  *A61B 5/145* (2006.01)
*A63B 24/00* (2006.01)

(21) Application number: **17167571.3**

(22) Date of filing: **21.04.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **TIEMANN, Christian Andreas
5656 AE Eindhoven (NL)**
• **LOWET, Dietwig Jos Clement
5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **FITNESS LEVEL PREDICTION DEVICE, SYSTEM AND METHOD**

(57)    The present invention relates to a fitness level prediction system and method to predict expected changes in fitness upon following a specific activity program. The physical load on a user and the corresponding fitness level are correlated within a model such that a simulation of said model leads to a prediction of a future fitness level. The method could also be used to evaluate the effect of activity programs on the fitness level, and hereby enable the tailoring of programs to reach a desired fitness goal.

FIG.1

**Description**

FIELD OF THE INVENTION

The present invention relates to a fitness level prediction device, a fitness level prediction system, a fitness level prediction method and a computer program.

BACKGROUND OF THE INVENTION

Cardiorespiratory fitness refers to the ability of a person's circulatory and respiratory systems to supply oxygen to skeletal muscles during sustained physical activity. Cardiorespiratory fitness is an important measure of human physiology with remarkable health, wellbeing, life quality, work ability, and performance-related associations. There are many benefits of good cardiorespiratory fitness. It can reduce the risk of heart disease, lung cancer, type-2 diabetes, stroke, and other diseases. Also, cardiorespiratory fitness helps to improve lung and heart condition, and increases feelings of wellbeing. It would therefore be very beneficial to track and predict changes in cardiorespiratory fitness over time. This information could, for instance, be used in health coaching programs. The present invention can be integrated in solutions for which monitoring, prediction and coaching related to cardio-fitness is relevant, e.g. coaching solutions for personal health and hospital to home.

Cardiorespiratory fitness is typically assessed by determining a person's VO2max, i.e. the maximum rate of oxygen consumption the person can achieve during sustained physical activity. Measuring VO2max directly is difficult and usually involves a graded exercise test on a treadmill or cycle ergometer in which exercise intensity is progressively increased while measuring the oxygen and carbon dioxide concentration of the inhaled and exhaled air. Therefore, in the past decades many indirect techniques and estimators have been developed to estimate VO2max in an easier and more consumer-friendly way. These methods provide an estimate of a user's current fitness level as represented by a user's VO2max.

A related field exists working on physical performance models. These models are typically focused on running and cycling athletes with the aim to predict optimal race performance. Here, physical performance is typically defined as the duration it takes to run/cycle a certain distance.

US 6135951 A discloses a personal fitness monitoring device and method for assessing the fitness of an individual as the individual exercises includes using a pedometer to determine and output data representing the locomotion of the individual. A heart rate monitor determines and outputs data representing the heart rate of the individual. A determination arrangement calculates the fitness of the individual as the individual exercises using personal data provided by the individual in combination with the data outputs of the pedometer and the heart rate without requiring a predetermined exercise regime.

SUMMARY OF THE INVENTION

It is an object of the present invention to provide a fitness level prediction device, system and method for predicting a future fitness level upon following a specific activity program.

In a first aspect of the present invention a fitness level prediction device is presented which comprises

- a physical load estimation unit configured to estimate the physical load on a user,
- a fitness level estimation unit configured to estimate the fitness level of the user,
- a fitness level model unit configured to provide a fitness level model generating a fitness level model output by correlating the estimated physical load on the user with the estimated fitness level of the user, and
- a simulating unit for simulating the fitness level model to predict a future fitness level of the user based on a planned future physical load on the user, i.e. on future physical activities of the user.

In a further aspect of the present invention a fitness level prediction system is presented which comprises

- a vital signs sensor for sensing a vital sign, and
- a fitness level prediction device

wherein the physical load estimation unit is configured to receive the sensed vital sign for estimating the physical load on the user and/or the fitness level estimation unit is configured to receive the sensed vital sign for estimating the fitness level of the user, and wherein a vital sign comprises one or more of heart rate, heart rate variability, sweat, bioimpedance, skin temperature, core body temperature, respiration rate, blood pressure, lactate concentration in blood, oxygen concentration of inhaled and exhaled air, carbon dioxide concentration of inhaled and exhaled air.

In a further aspect of the present invention a fitness level prediction method is presented which comprises

- estimating a physical load on a user,
- estimating the fitness level of the user,
- providing a fitness level model correlating the physical load on the user with the fitness level of the user, and
- simulating the fitness level model to predict a future fitness level of the user based on a past fitness level estimation and on a planned future physical load on the user. The future physical load on the user is estimated by evaluating an activity plan containing information required by the fitness level model, for example.

In yet a further aspect of the present invention, there is provided a computer program which comprises program code means to carry out the steps of the fitness level prediction method when said computer program is carried out on the computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed fitness level prediction method, computer program and medium have similar and/or identical preferred embodiments as the claimed device, in particular as defined in the dependent claims and as disclosed herein.

The present invention is based on the idea to provide a device and method to a user that predicts his future fitness level based on activities that he chooses in a training program. Current methods providing fitness level estimations are usually limited to estimating a person's VO2max level. These methods, however, merely estimate the person's current fitness level. The present invention particularly aims to predict changes in fitness level, which is an interesting parameter for a large population, in particular not only athletes. The proposed approach does not only take past fitness estimations into account in order to simulate future fitness levels, but it rather incorporates information related to physical activity a person plans to do, i.e. planned future loads. These may be provided in form of an activity plan which contains information concerning the expected intensity level and the expected duration of a training session as well as the gender of the user, for example. By introducing a fitness level model correlating the current physical load on a user with a current fitness level estimation it is possible to predict future fitness levels by simulating said model under parameter input concerning planned activities, i.e. planned (future) physical loads, of the user.

Typically, for health programs, such as "DirectLife", physical activity targets are set up in advance. The present invention has the advantage that it can integrate this information by simulating the cumulative effect of multiple planned training sessions on fitness. It can be used to evaluate the expected effect of different physical activity programs on fitness and thereby aid users and coaches to select or design an optimal program to reach a desired fitness goal. In this respect, the present invention is also suited to evaluate the impact on fitness when deviating from a planned program, e.g. because of illness, an injury, holiday, lack of motivation etc. This also applies to the build-up of fatigue and overtraining, which can be taken into account in the present invention as well.

The simulation may be improved by taking into account personal data of the user, such as gender, and/or information on vital signs of the user, physical activity data and/or environmental data. Hereby, a vital sign may comprise one or more of heart rate, heart rate variability, sweat, bioimpedance, skin temperature, core body temperature, respiration rate, blood pressure, lactate concentration in blood, oxygen concentration of inhaled and exhaled air, carbon dioxide concentration of inhaled and exhaled air. Personal data comprises of gender, age, weight, resting heart rate, maximal heart rate, heart rate zones, type of physical activities, duration of physical activities, intensity of physical activities and activity plans. Physical activity data comprises one or more of speed data, duration data, strength training data and pedometer data, and environmental data comprises one or more of altitude data, barometric data, weather data.

In an embodiment of the fitness level prediction device, the fitness level model may be configured to use the estimated physical load as input for generating a fitness level model output. This fitness level model output is then calibrated by correlation with the estimated current fitness level of the user. The kind of fitness level model output depends on the fitness level model chosen.

In an embodiment of the fitness level prediction device, the fitness level model output of the fitness level model is generated by balancing positive training effects and negative training effects of the physical load. Hence, the model takes a training load as input and contains two antagonistic components capturing the positive effects, termed PTE, and negative effects, termed NTE, of training on physical performance. That is, the fitness level model output is given by the difference between PTE and NTE. The fitness level model output is named 'performance' here following the impulse-response model of Banister et al. (Banister EW, MacDougall JD, Wenger HA, et al. Modeling elite athletic performance: physiological testing of the high-performance athlete. Campaign (IL): Human Kinetics Books; 1991: 403-25)

In an embodiment of the fitness level prediction device the fitness level model unit may further comprise a filtering unit configured to filter the fitness level model output of the fitness level model. The reason for this is that the physical performance of a person can be very dynamic and fluctuate significantly on short time scales. For instance, if one had a very intensive training session today, the maximal reachable performance of tomorrow will probably be much lower. Moreover, physical performance is also affected by many other factors including weather conditions, terrain, altitude, type of clothes and shoes, etc. The filtering ensures the trajectory of estimated future fitness levels to be smooth.

In an embodiment of the fitness level prediction device, the fitness level model unit is configured to calibrate the fitness level model by minimizing the difference between the, possibly filtered, fitness level model output and the estimated fitness level of a user. To be more specific, model parameter values are determined by using a least-squares estimation technique. This ensures that the model predictions of a person's fitness level are as close to the real fitness level as possible. Since the model calibration does not only take place once but rather takes place repeatedly with every training session of the user the model gets updated permanently and its fitness level predictions get more accurate in the course of usage.

In another embodiment of the fitness level prediction device, the fitness level estimation unit is configured to estimate the fitness level of a user by determining the user's fitness level marker during a physical activity, wherein the fitness level marker can be any one of the user's maximum rate of oxygen consumption, VO2max; peak oxygen consumption, VO2peak; exercise capacity in metabolic equivalent of task; anaerobic threshold; lactate threshold; ventilatory threshold. Hence, the fitness level estimation of the fitness level prediction device is not only limited to VO2max values indicating a person's fitness.

For example, VO2peak is the highest value of VO2 attained during a test. It does not necessarily define the highest value attainable by the person, which would be the VO2max. The term VO2peak is for instance used when certain criteria for a VO2max test are not met, e.g. observing a VO2 "plateau", although the person worked as hard as he/she could. A plateau in the VO2 response to ramp-type incremental test is not consistently seen in certain patient populations such as patients with pulmonary-mechanical abnormalities.

The fitness level of a person is also linked to the exercise capacity, i.e. the maximum amount of physical exertion a person can sustain, typically expressed in metabolic equivalent (MET). For instance, walking is about 3 MET, jogging about 6 MET, etc. MET is linked to oxygen consumption, where 1 MET = 3.5 ml O2/kg/min.

Another marker of fitness is the anearobic/lactate threshold, which refers to the exercise intensity at which the blood concentration of lactate begins to increase exponentially. During low intensity exercise, blood lactate remains at or near to resting levels. As exercise intensity increases there comes a break point where blood lactate levels rise sharply. Regular endurance exercise can lead to an increase of the threshold. Linked to the lactate threshold is the ventilatory threshold, which refers to the point during exercise at which the ventilation rate starts to increase at a faster rate than VO2. The threshold typically lies at exercise intensities between 50% and 75% of VO2max.

In another embodiment of the fitness level prediction device, the physical load estimation unit is configured to estimate the physical load on a user by evaluating a heart rate of the user and a duration of a physical activity of the user, and the simulating unit is configured to estimate a future physical load on the user by evaluating a planned future duration of a physical activity and a planned future intensity of the physical activity.

In order to estimate the physical load also other vital signs and personal data may be taken into account. In particular, physical activity data and environmental data may be used as well. This ensures a precise capturing of a person's physical load by taking into account not only one isolated parameter itself but by taking into account said parameter in the light of other parameters that can have influence on said parameter. The same applies to the estimation of the future physical load which may be derived from other personal data and/or physical activity data and which may be improved by means of the user's vital signs and/or environmental data.

The physical load of a training session is a combination of how intense and how long a training was. Many factors that make a training more or less intense are directly reflected in the heart rate, for example. Climbing a hill is more intense than walking on flat ground, running in windy conditions is more intense compared to windless conditions, running when you gained some kilos is more intense than when you weigh less, etc. All these effects will also be visible in the heart rate. Determining the fitness level it is more complicated. Existing methods to estimate one's current fitness level usually make use of heart rate data (see e.g., white paper from Firstbeat Technologies Ltd. Automated Fitness Level (VO2max) Estimation with Heart Rate and Speed Data, 2014). However, the heart rate is influenced by many factors, which could impact the VO2max estimation. For example, the heart rate is typically higher when the environmental temperature is higher, e.g. for cooling the body. Also, the heart rate at a certain running speed is higher when one is climbing, or when there are windy conditions. This could lead to an underestimation on one's fitness level. The VO2max estimation could be corrected by taking into account this additional information.

Some vital signs give more insights into how intense a training session was, e.g., lactate concentration gives an indication of muscular fatigue. Respiration rate also typically increases with training intensity.

In another embodiment of the fitness level prediction device, the physical load estimation unit is configured to estimate the physical load by using the training impulse method, TRIMP, based on a heart rate parameter, a duration of a physical activity and gender. Here, a daily training load, for example, is calculated by summing all individual TRIMP values of the respective day. Different activities like walking, running, and cycling, for instance, are recognized real-time by analyzing accelerometer data, for example, as is known from multiple smartphone apps and the corresponding heart rate data of these activity periods are extracted and then converted into a TRIMP value. Also for future planned training sessions a TRIMP value can be estimated, given a planned duration and intensity of the training. Being an established method, the incorporation of TRIMP in the present invention ensures a reliable estimation of training load values. Furthermore, the TRIMP method only uses the three mentioned parameters, which are convenient to determine.

In yet another embodiment of the fitness level prediction device, the fitness level model unit is configured to provide one or more of a performance model, a fitness-fatigue model, an impulse-response model and a regression model as fitness level model. In particular this includes all models developed by Banister et al. and all models derived from Banister's work developed by other researchers.

The fitness level prediction system may generally be any system comprising a vital signs sensor for sensing a vital sign and a fitness level prediction device, wherein the physical load estimation unit is configured to receive the sensed vital sign for estimating the physical load on a user and/or the fitness level estimation unit is configured to receive the sensed vital sign for estimating the fitness level of the user, and wherein a vital sign comprises one or more of heart rate, heart rate variability, sweat, bioimpedance, skin temperature, core body temperature, respiration rate, blood pressure, lactate concentration in blood, oxygen concentration of inhaled and exhaled air, carbon dioxide concentration of inhaled and exhaled air. This enables the physical load estimation unit and the fitness level estimation unit to estimate the user's physical load and the user's fitness level, respectively, based on a vital sign.

In another embodiment, the disclosed fitness level prediction system may further comprise a physical activity sensor configured to sense physical activity of a user, wherein the physical load estimation unit is configured to receive physical activity data for estimating the physical load on a user and/or the fitness level estimation unit is configured to receive physical activity data for estimating the fitness level of the user, wherein physical activity data comprises one or more of speed data, duration data, strength training data, pedometer data and acceleration data. This enables the physical load estimation unit and the fitness level estimation unit to estimate a user's physical load and the user's fitness level, respectively, based on physical activity data.

In a further embodiment, the fitness level prediction system further comprises a user interface configured to receive personal data and/or environmental data, wherein the simulating unit is configured to receive personal data and/or environmental data for simulating the fitness level model and for predicting a future fitness level of a user and wherein the physical load estimation unit is configured to receive personal data and/or the environmental data for estimating the physical load on the user, wherein personal data comprises one or more of gender, age, weight, resting heart rate, maximal heart rate, a heart rate zone, a type of physical activity, a duration of a physical activity, an intensity of a physical activity, an activity plan, and wherein environmental data comprises one or more of altitude data, barometric data, weather data. The user interface may also provide data concerning vital signs of the user as well a physical activity data as defined above.

BRIEF DESCRIPTION OF THE DRAWINGS

These and other aspects of the invention will be apparent from and elucidated with reference to the embodiments described hereinafter. In the following drawings

Fig. 1 shows a schematic diagram of an embodiment of a fitness level prediction device according to the present invention,
Fig. 2 shows a schematic diagram of an embodiment of fitness level model unit according to the present invention,
Figs. 3A and 3B show the relationship between physical load, positive and negative training effects and the fitness level model output of the fitness level model in case of the impulse-response model from Banister et al.,
Fig. 4 shows a diagram illustrating an embodiment of a method for predicting a future fitness level using a fitness level prediction method according to the present invention, and
Fig. 5 shows a schematic diagram of an embodiment of a fitness level prediction system according to the present invention.

DETAILED DESCRIPTION OF THE INVENTION

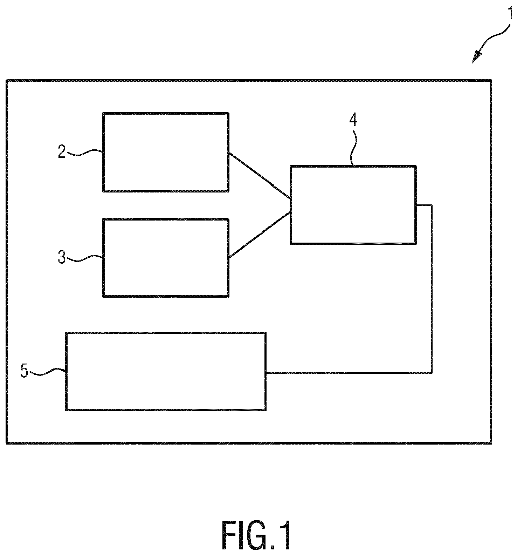

Fig. 1 shows a schematic diagram of an embodiment of a fitness level prediction device 1 according to the present invention. The fitness level prediction device 1 comprises a physical load estimation unit 2, a fitness level estimation unit 3, a fitness level model unit 4 and a simulating unit 5.

Both the physical load estimation unit 2 and the fitness level estimation unit 3 process data corresponding to vital signs, physical activity, personal information and/or environmental information. The physical load estimation unit 2 uses said data for estimating a physical load on a user, while the fitness level estimation unit 3 uses said data for estimating a fitness level of the user. Furthermore, both the physical load estimation unit 2 and the fitness level estimation unit 3 may pass the estimated physical load and the estimated fitness level, respectively, on to the fitness level model unit 4. The fitness level model unit 4 is configured to provide a fitness level model correlating these data. The simulating unit 5 then simulates the fitness level model in order to predict a future fitness level of the user. This prediction may also be based on a past fitness level estimation and on a planned future physical load on the user. Optionally, personal data of the user and/or environmental data may be used in addition.

There exist various methods to quantify the physical load of a training session. Typically these are based on physiological parameters like oxygen consumption, lactate concentration, or heart rate. A widely-used metric to quantify the physical load is called Training Impulse Method (TRIMP), which is based on heart rate parameters, training duration, and gender. The TRIMP is calculated as follows:

$$TRIMP = D * \Delta HR_{ratio} * Y,$$

wherein

$$\Delta HR_{ratio} = \frac{HR_{ex} - HR_{rest}}{HR_{max} - HR_{rest}},$$

and $Y = 0.64e^{1.92\Delta HR}$ for males and $Y = 0.86e^{1.67\Delta HR}$ for females. $D$ is the duration of the training in minutes, $HR_{ex}$ is the average heart rate during the training session, $HR_{rest}$ is the resting heart rate, and $HR_{max}$ is the maximum heart rate of the user. The daily training load is calculated by summing all individual TRIMP values of the respective day. Also for future planned training sessions a TRIMP value can be estimated, given a planned duration and intensity of the training.

However, the present invention is not limited to the usage of the TRIMP. In principle any method to quantify training load can be used in the present invention.

There also exist various methods to estimate a person's current fitness level. An existing method concerns the one from Firstbeat (white paper from Firstbeat Technologies Ltd. Automated Fitness Level (VO2max) Estimation with Heart Rate and Speed Data, 2014). In brief, during a training session the relation between heart rate and running speed is determined. Next, the expected speed (Vmax) at the person's maximal heart rate is determined via extrapolation. The relation between running speed and oxygen consumption (VO2) is well-studied and follows a linear relation. This relation is subsequently used to estimate VO2max from Vmax.

The fitness level model provided by the fitness level model unit 4 can be one or more of a performance model, a fitness-fatigue model and an impulse-response model. In particular this includes all fitness level models developed by Banister et al. and all models derived from Banister's work developed by other researchers.

The simulating unit 5 simulates the fitness level model in order to estimate a future fitness level of the user. This estimation is represented by an extrapolation taking into account past fitness estimations. However, this estimation may also be based on future physical activities of the user and/or personal data of the user and/or environmental data. The steps are quite similar as in the model calibration part, in which the fitness level model output is calculated using TRIMP values of the performed training as input. In the simulator part the fitness level model output is calculated using the parameters obtained during calibration. The input is in this case a series of estimated TRIMP values based on training the persons plans to do. This can be done on a day resolution, for example. So if a user plans a training program of three weeks, 21 TRIMP values need to be estimated. On the days the person does not train, the TRIMP value will be zero. On training days an estimation has to be made of the TRIMP, based on the expected duration and the intensity of the training. In particular, $\Delta HR_{ratio}$ of the above mentioned TRIMP formula is linked to the training intensity. Depending on the planned training intensity an estimation of $\Delta HR_{ratio}$ can be made. For instance, a value of 0.5 of $\Delta HR_{ratio}$ represents a very low training intensity, whereas a value of 0.9 of $\Delta HR_{ratio}$ represents very high training intensity.

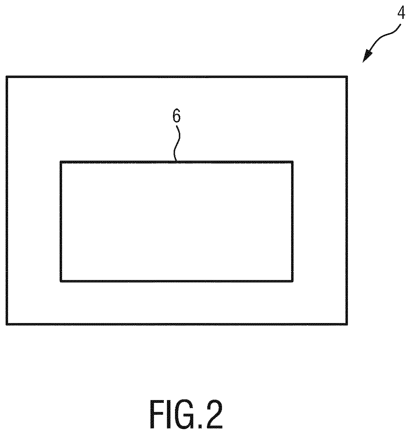

Fig. 2 shows a schematic diagram of an embodiment of fitness level model unit according to the present invention. The fitness level model unit 4 may comprise a filtering unit 6 configured to filter the output of the fitness level model.

The fitness level model output is filtered, e.g. using a moving average, before the model parameters are estimated. The reason for this is that the fitness level model output can change quite rapidly. This is because the fitness level model output is based on the physical load on a user. That is, if one ran a marathon today, the physical load of the next day will probably be much lower, although the overall fitness level will not change in one day time. In fact, the fitness level, for example reflected by the VO2max, is more stable and changes only gradually over time. The filtering provided by the filtering unit 6 is configured to smooth out the corresponding differences in the fitness level model output of the fitness level model.

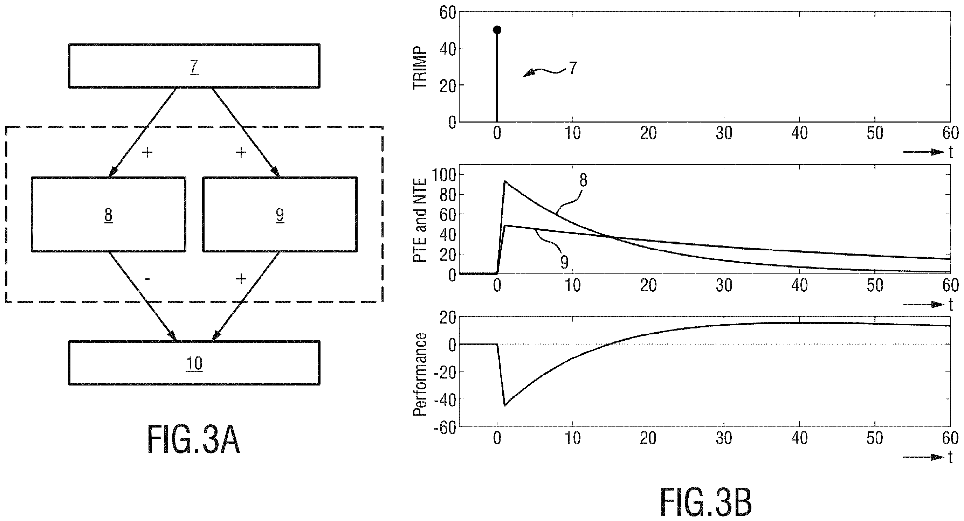

Figs. 3A and 3B show the relationship between physical load 7, negative training effects 8 and positive training effects 9 and the fitness level model output 10 of the fitness level model in case of the impulse-response model from Banister et al..

Fig. 3A shows a schematic overview of the impulse-response model from Banister et al.. The model takes the daily training load 7, i.e. the daily physical load 7 as input. A physical load 7 corresponding to other time periods is conceivable as well. The model contains two antagonistic components capturing the positive effects 9 (termed PTE) and negative effects 8 (termed NTE) of training on physical performance 10. Physical performance 10, i.e. the fitness level model output 10, is given by the difference between PTE 9 and NTE 8. Mathematically, PTE 9 and NTE 8 are given by a sum of training impulses, each modified by an exponential decay term:

$$p^n = p^* + k_1 \sum_{i=1}^{n-1} w^i e^{\frac{-(n-i)}{\tau_1}} - k_2 \sum_{i=1}^{n-1} w^i e^{\frac{-(n-i)}{\tau_2}}.$$

$p^*$ represents the basic performance, also called initial performance, which represents the performance level at start, basically an offset. We calibrate the model using the estimated fitness level. Hence, in our case this would be a VO2max offset, i.e., the VO2max of a person when he/she would be physically inactive.

The second term stands for positive training effects 9, while the third term stands for negative training effects 8. In brief, PTE is a collection of physiological changes due to training that increase the physical performance (for example improved vasculature), and NTE is a collection of physiological changes due to training that decrease the physical performance, for example muscle damage or muscular fatigue.

The dynamics of the model are determined by four parameters. $k_1$ and $k_2$ are gain factors determining the magnitude of the instantaneous response in PTE 9 and NTE 8, respectively, upon a training impulse. Parameters $\tau_1$ and $\tau_2$ determine how fast PTE 9 and NTE 8, respectively, decay exponentially over time.

Fig. 3B depicts an example model simulation of the dynamic response of PTE 9, NTE 8, and physical performance 10 components over a time period of 60 days upon a single training impulse. Here, the impulse-response model of Banister has been used as fitness level model. Typically, the PTE 9 that is gained in a training is of a moderate magnitude but is long lasting, whereas the NTE 8 that accumulates in a training is of high magnitude, but does not last very long. The graphs show clearly the impulse-response behavior of the Banister model.

The fitness level model output, as well as the PTE 9 and NTE 8 components, are dimensionless. Hence, the fitness level model output has to be scaled to a performance metric of interest, e.g., the duration to run/cycle a certain distance, or the maximal wattage one can achieve, etc. This can for instance be achieved by choosing specific values for parameters $k_1$, $k_2$, $\tau_1$ and $\tau_2$. The same applies if the fitness level model output shall be translated to a VO2max estimate, for instance.

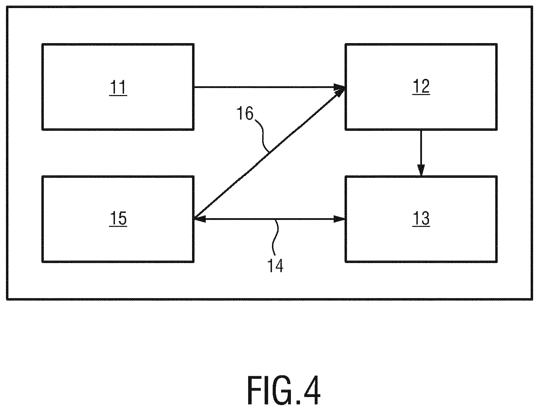

Fig. 4 shows a diagram illustrating an embodiment of a method for estimating a future fitness level using a fitness level prediction method according to the present invention.

The fitness level model 12 is configured to use the physical load estimation 11 of a certain time period as input for generating the fitness level model output 13. The fitness level model output 13 may then be filtered, however, this is not crucial for the present invention. In the next step, a correlation 14 between the fitness level model output 13 and the estimated fitness level 15 corresponding to the same time period takes place. Usually, a VO2max estimation as fitness level estimation 15 are used, however other fitness level markers are also conceivable to be used in the present invention. By minimizing the difference between the fitness level model output 13 and VO2max estimations, for example, a calibration 16 of the parameters of the fitness level model 12 is carried out. In particular, the determination of said values may be achieved using a least-squares optimization technique. The parameter estimation is repeated when new data, i.e. new physical load estimations 11 and fitness level estimations 15, are obtained. Hence, the dynamic characteristics of how a person responds to performed training are learned over time.

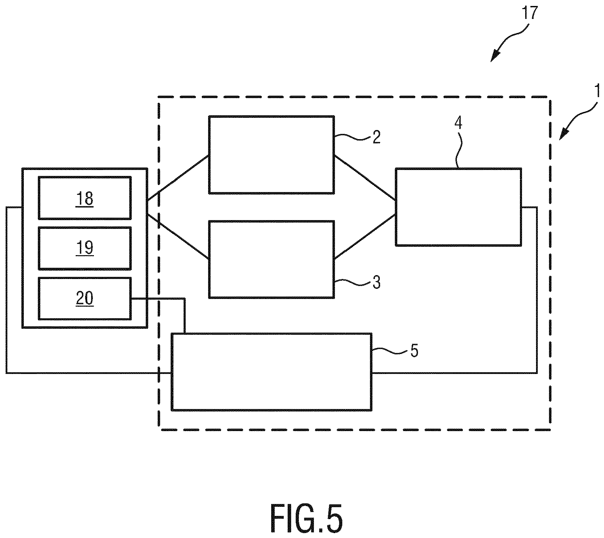

Fig. 5 shows a schematic diagram of an embodiment of a fitness level prediction system 17 according to the present invention. The system 17 comprises a fitness level prediction device 1 and further comprises a vital signs sensor 18, a physical activity sensor 19 and a user interface 20. The vital signs sensor 18 is configured to sense a vital sign of the user, wherein the physical load estimation unit 2 and/or the fitness level estimation unit 3 is configured to use the sensed vital sign for estimating the physical load and/or the fitness level, respectively. The physical activity sensor 19 is configured to sense a physical activity of the user, wherein the physical load estimation unit 2 and/or the fitness level estimation unit 3 is configured to use the sensed physical activity data for estimating the physical load and/or the fitness level, respectively. The user interface 20 is configured to receive personal data of the user and/or environmental data. Furthermore the user interface can receive data from external sources, such as VO2max measurement data obtained from lab measurements or other tests.

The physical load estimation unit 2 and/or the fitness level estimation unit 3 is configured to use said data for estimating the physical load and/or the fitness level, respectively. The physical load estimation unit 2 and the fitness level estimation unit 3 pass on the estimated physical load data 11 and the estimated fitness level data 15, respectively, to the fitness level estimation unit 4, which is configured to provide a fitness level model 12 linking both estimations to each other by using a model calibration 16. The simulating unit 5 simulates the calibrated fitness level model 12 using data from the user interface 20 to estimate a future fitness level of the user.

While estimating a physical load 7 and estimating a current fitness level are generally known in the current state of the art, correlating them within a model such that a fitness level can be predicted for the future is not known. The benefit of the correlation 14 is in the case of training programs wherein you can tell the participant in advance what his fitness would be if he followed a prescribed program given his current fitness level.

Furthermore, with each training session model calibration and simulation takes place repeatedly, optimizing the model further by adapting the parameters accordingly.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1.   A fitness level prediction device (1) comprising:

- a physical load estimation unit (2) configured to estimate the physical load (7) on a user,
- a fitness level estimation unit (3) configured to estimate the fitness level (15) of the user,
- a fitness level model unit (4) configured to provide a fitness level model (12) correlating the estimated physical load (7) on the user with the estimated fitness level (15) of the user, and
- a simulating unit (5) for simulating the fitness level model (12) to predict a future fitness level of the user based on a planned future physical load on the user.

2.   The fitness level prediction device (1) according to claim 1,
wherein the fitness level model (12) is configured to use the estimated physical load (7) as input for generating a fitness level model output (13).

3.   The fitness level prediction device (1) according to claim 2,
wherein the fitness level model output (13) is generated by balancing positive training effects (9) and negative training effects (8) of the physical load (7).

4.   The fitness level prediction device (1) according to claims 2 to 3,
wherein the fitness level model unit (4) comprises a filtering unit (6) configured to filter the fitness level model output (13) of the fitness level model (12).

5.   A fitness level prediction device (1) according to any one of the preceding claims,
wherein the fitness level model unit (4) is configured to calibrate the fitness level model (12) by minimizing the difference between the fitness level model output data (13) and the estimated fitness level (15) of the user.

6.   The fitness level prediction device (1) according to any one of the preceding claims,
wherein the fitness level estimation unit (3) is configured to estimate the fitness level (15) of a user by determining the user's fitness level marker during a physical activity, wherein the fitness level marker can be any one of the user's maximum rate of oxygen consumption, VO2max; peak oxygen consumption, VO2peak; exercise capacity in metabolic equivalent of task; anaerobic threshold; lactate threshold; ventilatory threshold.

7.   The fitness level prediction device (1) according to any one of the preceding claims,
wherein the physical load estimation unit (2) is configured to estimate the physical load (7) on a user by evaluating a heart rate of the user and a duration of a physical activity of the user, and
wherein the simulating unit (6) is configured to estimate a future physical load on the user by evaluating a planned future duration of a physical activity and a planned future intensity of the physical activity.

**8.** The fitness level prediction device (1) according to any one of the preceding claims,
wherein the physical load estimation unit (2) is configured to estimate the physical load (7) by using the training impulse method, TRIMP, based on a heart rate parameter, a duration of a physical activity and gender.

**9.** The fitness level prediction device (1) according to any one of the preceding claims,
wherein the fitness level model unit (4) is configured to provide one or more of a performance model, a fitness-fatigue model, an impulse-response model and a regression model as fitness level model.

**10.** A fitness level prediction system (17) comprising:

- a vital signs sensor (18) for sensing a vital sign, and
- a fitness level prediction device (1) as claimed in claim 1,

wherein the physical load estimation unit (2) is configured to receive the sensed vital sign for estimating the physical load (7) on a user and/or the fitness level estimation unit (3) is configured to receive the sensed vital sign for estimating the fitness level (15) of the user and wherein a vital sign comprises one or more of heart rate, heart rate variability, sweat, bioimpedance, skin temperature, core body temperature, respiration rate, blood pressure, lactate concentration in blood, oxygen concentration of inhaled and exhaled air, and carbon dioxide concentration of inhaled and exhaled air.

**11.** The fitness level prediction system (17) according to claim 10,
further comprising a physical activity sensor (19) configured to sense a physical activity of a user, wherein the physical load estimation unit (2) is configured to receive physical activity data for estimating the physical load (7) on the user and/or the fitness level estimation unit (3) is configured to receive physical activity data for estimating the fitness level (15) of the user, wherein physical activity data comprises one or more of speed data, duration data, strength training data, pedometer data and acceleration data.

**12.** The fitness level prediction system (17) according to any one of claims 10 to 11,
further comprising a user interface (20) configured to receive personal data and/or environmental data, wherein the simulating unit (5) is configured to receive personal data and/or environmental data for simulating the fitness level model (12) and for predicting a future fitness level of a user and wherein the physical load estimation unit (2) is configured to receive personal data and/or the environmental data for estimating the physical load (7) on the user, wherein personal data comprises one or more of gender, age, weight, resting heart rate, maximal heart rate, a heart rate zone, a type of physical activity, a duration of a physical activity, an intensity of a physical activity, and an activity plan, and
wherein environmental data comprises one or more of altitude data, barometric data, and weather data.

**13.** A fitness level estimation method comprising:

- estimating the physical load (7) ON a user,
- estimating the fitness level (15) of the user,
- providing a fitness level model (12) correlating the physical load (7) on the user with the fitness level (15) of the user, and
- simulating the fitness level model (12) to predict a future fitness level of the user based on a planned future physical load on the user.

**14.** A computer program comprising program code means for causing a computer to carry out the steps of the fitness level prediction method as claimed in claim 13 when said computer program is carried out on the computer.

FIG.1

FIG.2

FIG.3A

FIG.3B

EP 3 391 809 A1

FIG.4

FIG.5

Europäisches
Patentamt

European
Patent Office

Office européen
des brevets

# EUROPEAN SEARCH REPORT

Application Number

EP 17 16 7571

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/261776 A1 (SKIBA PHILIP [US]) 23 October 2008 (2008-10-23) * abstract; figures 1-8 * * paragraphs [0004] - [0010], [0011] - [0014], [0023] - [0090] * ----- | 1-14 | INV. A61B5/00 A61B5/083 A61B5/08 A61B5/11 A61B5/0205 |
| X | US 2015/327804 A1 (LEFEVER JORIS [BE] ET AL) 19 November 2015 (2015-11-19) * abstract; figures 1-5 * * paragraphs [0001], [0016] - [0030], [0041] - [0082] * ----- | 1-14 | A61B5/024 A61B5/053 A61B5/145 ADD. A63B24/00 |
| X | US 2009/069156 A1 (KURUNMAEKI VELI-PEKKA [FI] ET AL) 12 March 2009 (2009-03-12) * abstract; figures 1-13 * * paragraphs [0061] - [0147] * ----- | 1-14 | |

TECHNICAL FIELDS
SEARCHED     (IPC)

A61B
A63B

The present search report has been drawn up for all claims

| Place of search | Date of completion of the search | Examiner |
|---|---|---|
| The Hague | 5 October 2017 | Carta, Riccardo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 16 7571

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2017

| Patent document cited in search report | | Publication date | Patent family member(s) | | Publication date |
|---|---|---|---|---|---|
| US 2008261776 | A1 | 23-10-2008 | NONE | | |
| US 2015327804 | A1 | 19-11-2015 | AU | 2013220945 A1 | 02-10-2014 |
| | | | EP | 2815344 A1 | 24-12-2014 |
| | | | US | 2015327804 A1 | 19-11-2015 |
| | | | WO | 2013120151 A1 | 22-08-2013 |
| US 2009069156 | A1 | 12-03-2009 | EP | 1993681 A1 | 26-11-2008 |
| | | | FI | 20085920 A | 29-09-2008 |
| | | | US | 2009069156 A1 | 12-03-2009 |
| | | | WO | 2007099206 A1 | 07-09-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6135951 A

**Non-patent literature cited in the description**

- **BANISTER EW ; MACDOUGALL JD ; WENGER HA et al.** Modeling elite athletic performance: physiological testing of the high-performance athlete. Human Kinetics Books, 1991, 403-25